# EUROPEAN PATENT APPLICATION

(11) **EP 1 304 633 A1**
(43) Date of publication of application: **23.04.2003**
(21) Application number: 01934535.4
(22) Date of filing: 04.06.2001
(51) Int. Cl.: G06F 17/60

(54) **MEASUREMENT DEVICE, AND MEASURED DATA TRANSMITTING METHOD**

(30) Priority: 02.06.2000 JP 2000205906
(71) Applicant: ARKRAY, Inc., Kyoto-shi, Kyoto 601-8045 (JP)
(72) Inventor: DOI, Shigeru, c/o Arkray, Inc., Kyoto-shi, Kyoto 601-8045 (JP)
(74) Representative: Piésold, Alexander J.
(86) International application number: JP0104714
(87) International publication number: WO01093143

(57) **Abstract**

It is an object to provide a measuring device capable of collectively transmitting the measured data of a plurality of measuring devices to a data gathering device without requiring complex operations. A compact blood glucose measuring device (1) stores its measured data (data measured by the measuring device) and measured data (data measured by other measuring devices) that are transmitted from other measuring devices, such as a sphygmomanometer (6) and a urine analysis device (7), and when sending the measured data to a data gathering device (5), correlates the device ID and/or patient ID stored in an ID storage portion with the measured data obtained by the device and the measured data obtained by other devices and transmits.

## Description

### Technical Field

The present invention relates to a measuring device and a measured data transmission method with which patients can measure vital sign data such as their blood glucose level, blood pressure, pulse, temperature, and body weight from their homes and transmit the results of this measurement to a computer located in a medical institution, for example, via a public communications line or the like.

### Background Art

Conventionally, home health management devices are known that receive the measured data of, for example, an electrocardiograph for taking an electrocardiogram, a sphygmomanometer for measuring blood pressure and pulse, or a blood glucose meter for measuring glucose levels in the blood, record the measured data, and add data that identifies the measured person to the measured data and transmit the measured data to a hospital or the like. With such home health management devices, generally, a single device is capable of managing the personal data of a plurality of measured persons. This requires recognition of the measured person, and thus prompts instructing users to input data for specifying the measured person are displayed on a screen, or the device is capable of reading a magnetic card or the like onto which a personal ID has been stored.

Here, the home health management device disclosed in JP 2000-83907A illustratively serves as one example of a conventional home health management device, and the procedure through which it is used is explained. First, the power is turned on to start up the device and a screen for specifying the user appears. The names of users that have been registered in the device are displayed on the screen. From these names, the user selects the name of the person who will use the device to perform a measurement. Users who are using the device for the first time input their name via a touch panel to register as new users.

Although not disclosed in this Laid-Open Patent Application, conventional home health management devices also may include a device for identifying users by magnetic card or the like, in which case a magnetic card is swiped through a magnetic card reader attached to the measuring device so as to specify the ID of the user to the device.

Next, the display on the screen of the device switches to a screen for selecting an action to perform next. Here, if "Measure/Record" is selected, then the display switches to a screen for selecting what to measure. The user then selects an item to be measured from the screen and performs the measurement. For example, if an electrocardiogram is to be performed, then the user selects "Electrocardiogram", retrieves the electrocardiograph from the device, and performs an electrocardiogram. After taking the electrocardiogram, the patient then, for example, selects Blood Pressure" from the screen, retrieves the sphygmomanometer from the device, and measures his blood pressure. When measurement of the blood pressure is over, the patient for example selects "Blood Glucose Level,'' retrieves the blood glucose level measuring device from the device, and measures his blood glucose level.

The electrocardiograph, the sphygmomanometer, and the blood glucose level measuring device are cordless devices, and are configured so as to transmit the measured data to the home health management device via infrared light communication, for example, after the user has finished measurement with the devices. Using a similar procedure, the user performs a measurement of each item and stores the measured results in the home health management device. When the measurement is over, the user presses "End" on the screen. This returns the display screen to the "Select an Action" screen.

To view the measured results, a user can select "Display" on the "Select an Action" screen, however, this action is not described here. From the same screen, when "Telephone" is selected, the display screen turns into a screen that reads "Select Where to Call," and measured data transmission destinations such as hospitals that are registered in the device are displayed. When a measured data transmission destination is selected from among these, the home health management device carries out transmission of the measured data to the destination that is selected.

Also, in addition to the device that is disclosed in the above-mentioned Laid-Open Patent Application, conventionally, personal measuring devices that are home measuring devices such as sphygmomanometers or compact blood glucose. measuring devices and that also have a function for outputting data are commercially available. With these personal measuring devices, users can incorporate the measured data into a personal computer in their home and manage the measured data. In addition, by connecting these personal measuring devices to a data gathering device in the hospital when making a trip to the hospital and transmitting the measured data, physicians are able to utilize the measured data when making a diagnosis.

In particular, among compact blood glucose measuring devices there are known devices where the measuring device itself stores individual ID numbers, and that, when transmitting the results of measurements to the data gathering device, correlate the device ID with the measured data. Thus, the data gathering side identifies users by their device IDs and adds the measured results of users to data that have been stored to date as the measured results of that user, so that it can store new data.

As described above, one premise of home health management devices is that the same device will be shared by a plurality of individuals, such as several members of a family. Thus when measurements are to be taken, it is first necessary to establish who will be performing measurements, and an operation for identifying the user is required prior to starting measurements.

Also, home health management devices include not only a function for sending and receiving data but also numerous other functions, such as a function for displaying past measured data in a graph. Accordingly, the use of these functions requires various settings to be performed, which necessitates complicated operations, and this has led to the problem of the device being difficult for elderly persons to use.

With compact blood glucose devices having a communications function, the operation itself is easy. For example, with each of the devices, data transmission can be performed simply by connecting a communications cable or by connecting a communications cable and pressing a data transmission button. However, when managing data for a plurality of measured data items, such as "Glucose Level," "Blood Pressure," and "Pulse," some devices did not include a clock function, and thus to correlate and store what measured data were measured at the same time as other data, either the measured results had to be transmitted to the data gathering device immediately after measurement or the troublesome operation of editing the data with the data gathering device after reception of the data and correlating the data became necessary.

The present invention was arrived at in light of the above-mentioned problems, and it is an object of the present invention to provide a measuring device and a measured data transmission method with which data can be transmitted to a data gathering device by a simple operation.

### Disclosure of Invention

To achieve the foregoing objects, a measuring device of the present invention includes a storage portion for storing measured results, a reception portion for receiving data from another measuring device, a transmission portion for transmitting data to a data gathering device, and an identifier storage portion for storing identifiers for identifying attributes of measured data, wherein the storage portion has regions for respectively storing measured data obtained by the measuring device and measured data of the same measured person obtained by another measuring device, which are received by the reception portion, and wherein at least one of the measured data obtained by the measuring device and the measured data obtained by another measuring device stored in the storage portion are correlated with at least one identifier stored in the identifier storage portion and are transmitted to the data gathering device from the transmission portion.

According to this measuring device, measured data obtained by a plurality of types of measuring devices can be transmitted collectively to a data gathering device. Moreover, an identifier stored in the identifier storage portion is correlated with the measured data and the measured data are transmitted, and thus there is no need for users to add information pertaining to whom the measured data belong. Consequently, a measuring device with which measured data can be transmitted to the data gathering device through a simple operation and which is easy to use for users unaccustomed to operating machines, such as elderly persons, can be provided.

It is preferable that the measuring device further is provided with a clock portion indicating date and time, and that when the measured data obtained by another device received by the reception portion do not include measured date and time information, information on date and time obtained from the clock portion are correlated with the measured data obtained by the other device, and the measured data are stored in the storage portion.

According to this configuration, information on the date and time of reception can be assigned to measured data that are received from other measuring devices that do not have a clock function. Thus, if measured data from a particular measuring device are received immediately after a measurement is made, then the time when the measured data were measured can be identified with reasonable accuracy, even if the other measuring device does not have a clock function.

In addition, to achieve the foregoing objects, a measured data transmission method according to the present invention includes gathering, in a main measuring device to which identifiers for identifying measured persons are stored, measured data obtained by another measuring device for that measured person and storing the measured data in a storage portion of the main measuring device, and correlating the identifiers with at least one of the measured data obtained by the main measuring device and the measured data of the same measured person obtained by another measuring device, and transmitting the measured data from the main measuring device to a data gathering device.

Thus, users do not have to add information pertaining to whom the measured data belong, and the measured data can be transmitted to a data gathering device through a simple operation.

### Brief Description of Drawings

Fig. 1 is a block diagram showing the configuration of a measuring device according to an embodiment of the present invention.
Fig. 2 is a diagram showing a configuration of a system in which the measuring device according to the present invention is connected to other measuring devices and a data gathering device.
Fig. 3 is a diagram showing an example of measured data obtained by the measuring device.
Fig. 4 is a diagram showing an example of measured data obtained by another measuring device.
Fig. 5 is a diagram showing a further example of measured data obtained by another device.
Fig. 6 is a diagram showing how the time data are inserted into the data shown in Fig. 5.
Fig. 7 is a diagram showing an example of data transmitted from the measuring device to the data gathering device.

### Best Mode for Carrying Out the Invention

Hereinafter, an embodiment of the present invention is described in detail with reference to the drawings.

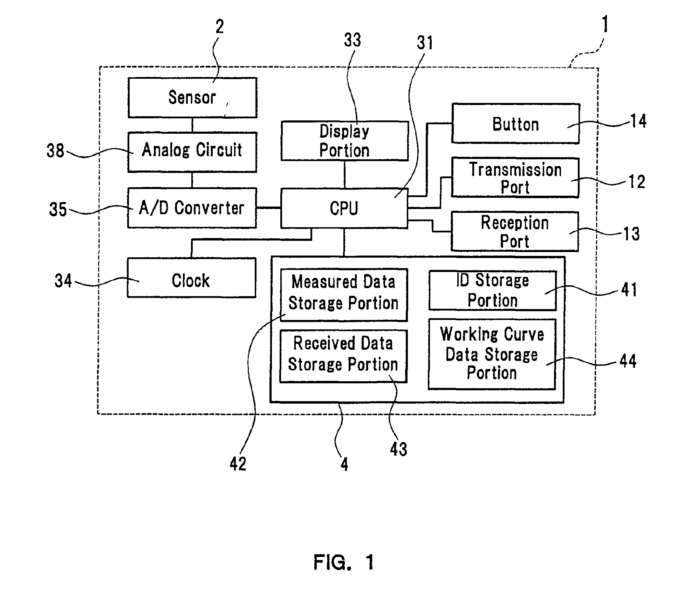

Fig. 1 is a block diagram showing the internal configuration of a compact blood glucose measuring device 1. The compact blood glucose measuring device 1 is provided with a CPU 31 serving as a control portion, to which are connected a storage portion 4, a display portion 33, a clock 34, an A/D converter 35, a transmission port 36 for transmitting data to the outside, and a reception port 37 for receiving data from the outside. The storage portion 4 includes an ID storage portion 41, a measured data storage portion 42, a received data storage portion 43, and a working curve data storage portion 44.

It should be noted that a sensor 2 is used by inserting it into a later-described sensor insertion portion on the compact blood glucose measuring device 1 and that blood from users is placed on the sensor 2. Accordingly, one sensor 2 is used per measurement and once the measurement is over, the sensor 2 is discarded, that is, the sensor 2 is a so-called disposable sensor.

The device ID and patient IDs are stored in the ID storage portion 41. The device ID is a unique ID assigned to each compact blood glucose measuring device 1 during manufacturing, and is stored in advance in the ID storage portion 41. Patient IDs are unique ID numbers assigned to each patient by the medical institution providing the patients with the compact blood glucose measuring device 1, and by performing a predetermined registration operation, the medical institution or the patient can store patient IDs in the ID storage portion 41.

With the compact blood glucose measuring device 1, the output from the sensor 2 is amplified by an analog circuit 38, converted into digital data by the A/D converter 35 and sent to the CPU 31, and converted into a blood glucose level based on working curve information stored in the working curve data storage portion 44 in advance. The obtained blood glucose level is displayed on the display portion 33, and is correlated with the time of the clock 34 and stored in the measured data storage portion 42. A button 14 can be pressed when the power of the compact blood glucose measuring device 1 is off so as to turn on the power of the device. In addition, the button 14 can be pressed when the power is on so as to transmit the data stored in the storage portion 4 via the transmission port 36.

Next, an example of the configuration and the operation of a system including the measuring device according to this embodiment is described.

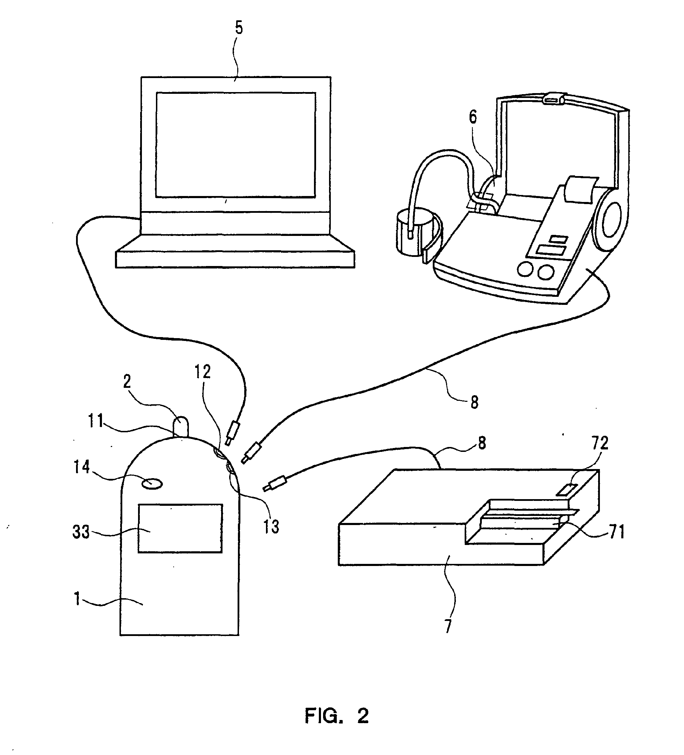

Fig. 2 is a diagram showing a system in which the measuring device of this embodiment is connected to another measuring device and a data gathering device.

As shown in Fig. 2, by connecting a cable of a data gathering device 5 to a transmission port 12, the compact blood glucose measuring device 1 can transmit measured data to the data gathering device 5. Also, by connecting a cable of another measuring device 6 to a reception port 13, the compact blood glucose measuring device 1 can take in measured data that are obtained by the other measuring device 6.

The operation procedure is described below.

The user first attaches the sensor 2 to a sensor insertion portion 11 of the compact blood glucose measuring device 1, and then places a small amount of blood drawn from his finger tip, for example, onto the sensor 2 and measures the blood glucose level of the blood. In a normal measurement, the compact blood glucose measuring device 1 is turned on by inserting the sensor 2 into the sensor insertion portion 11. Then, the compact blood glucose measuring device 1 starts the measurement when it detects that blood has been applied to the sensor 2. The measurement results are displayed on the display portion 33 approximately 30 seconds later, and measurement time data obtained from the clock 34 are correlated with the measured data and the measured data are stored in the storage portion 4.

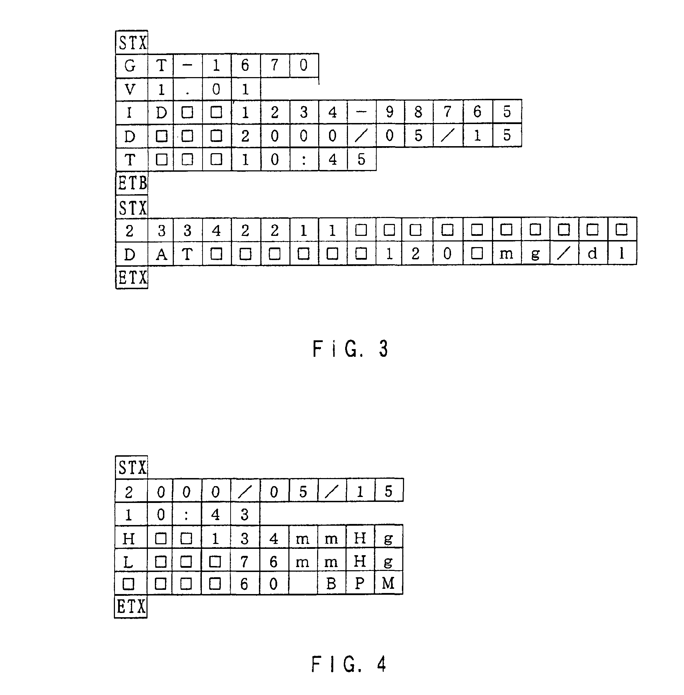

Data stored in the storage portion 4 are divided into first and second blocks. The first block includes data relating to the type name, the software version number, the device ID, the measurement date, and the measurement time. The second block includes the patient ID and the measured data. An example of these data is shown in Fig. 3.

As shown in Fig. 3, the data start at the start code "STX" and the end of one block is expressed by the block end code "ETB" and the end of the data is expressed by the end code "ETX." Each box in Fig. 3 represents a single character, and empty squares within the boxes indicate blanks.

In the example of Fig. 3, the type name is GT-1670, the software version number is V1.01, the device ID is 1234-98765, the measurement date is 2000/05/15, the measurement time is 10:45, the patient ID is 23342211, and the measured blood glucose level is 120 mg/dl.

Next, the user performs a measurement of items other than the blood glucose level. This embodiment is described with respect to a sphygmomanometer and a urine analysis device, but analysis devices such as heart rate meters and oxygen saturation meters can also be used. The compact blood glucose measuring device 1 is programmed to automatically shut off once a minute or more has passed since the end of a measurement, and if the power is off when other items are to be measured, then the user can press the button 14 to turn on the power.

Here, the user turns on the power of the sphygmomanometer 6 and connects the output port of the sphygmomanometer 6 to the reception port 13 of the compact blood glucose measuring device 1 by a cable 8. The user then presses the measurement start button (not shown) of the sphygmomanometer 6 and measures the systolic and diastolic pressure values. After measurement of the blood pressure is over, the user presses the transmit button (not shown) of the sphygmomanometer 6 to output the measured data from the output port of the sphygmomanometer 6. The measured data are received by the reception port 13 of the compact blood glucose measuring device 1. The data are transmitted in a text format.

Fig. 4 shows an example of measured data that are transmitted from the sphygmomanometer 6 to the compact blood glucose measuring device 1. The measured data of the sphygmomanometer 6 include data pertaining to the measurement date, the measurement time, the systolic pressure value, the diastolic pressure value, and the pulse rate. In the example of Fig. 4, the measurement date is 2000/05/15, the measurement time is 10:43, the systolic pressure value is 134 mmHg, the diastolic pressure value is 76 mmHg, and the pulse rate is 60 BPM.

Next, the user performs a urine analysis with a urine analysis device 7. Here, four items are analyzed: urinary glucose (GLU), urinary protein (PRO), pH, and urinary blood (BLD). The user puts a sample of his urine in a paper cup and brings the paper cup to a measurement spot. Then, the user connects the data output port on the rear surface of the urine analysis device 7 to the reception port 13 of the compact blood glucose measuring device 1 via a cable (not shown).

The user retrieves a testing paper, to which are attached testing paper pads for the four items, from a storage vessel, brings the testing paper into contact with the urine sample in the paper cup, and presses a start button 72 of the urine analysis device 7. The testing paper is set onto a testing paper rest 71 of the urine analysis device 7, and after approximately 30 seconds have passed, the testing paper rest 71 is drawn into the urine analysis device 7. The urine analysis device 7 measures the reflectance of the testing paper that has been drawn in, converts the reflectance into the concentration of each item, and outputs the results from the output port. The urine analysis device 7 is not provided with a clock function, and thus there are no data on the measurement time. That is, only the measurement results for each item are output from the urine analysis device 7.

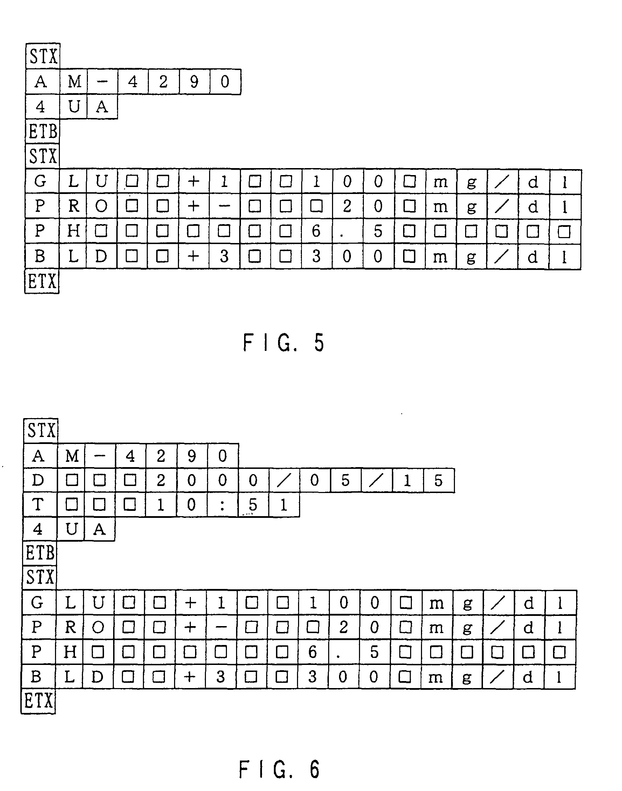

An example of the measured data obtained by the urine analysis device 7 is shown in Fig. 5. The data are divided into first and second blocks, with the first block including, as measurement information, the device name (AM-4290) and information on the testing paper (4UA) that is used. The second block includes the item name and the measurement results for each measured item. The measurement results include qualitative results and semi-quantitative values, depending on the item. In the example shown in Fig. 5, for GLU the qualitative result is +1 and the semi-quantitative value is 100 mg/dl, for PRO the qualitative result is +- and the semi-quantitative value is 200 mg/dl, the pH is 6.5, and for BLD the qualitative result is +3 and the semi-quantitative value is 300 mg/dl.

When the compact blood glucose measuring device 1 receives the measured data from the urine analysis device 7, data pertaining to the measured date and time are not included in these measured data, as was mentioned before, and thus data on the date and time are received from the clock 34 of the compact blood glucose measuring device 1 and correlated with the measured data, and the measured data are then stored in the received data storage portion 43. An example of the data stored in the received data storage portion 43 is shown in Fig. 6. As shown in Fig. 6, data on the date and time obtained from the clock 34 are inserted into the first block of the data received from the urine analysis device 7.

The procedure for transmitting data from the compact blood glucose measuring device 1 to the data gathering device 5, which is a personal computer or the like, is described next.

The user connects the transmission port 12 and the data gathering device 5 via a communications cable. A data reception program is activated in advance on the data gathering device 5 side. Data are transmitted to the data gathering device 5 from the compact blood glucose measuring device 1 when the user presses the button 14 of the compact blood glucose measuring device 1.

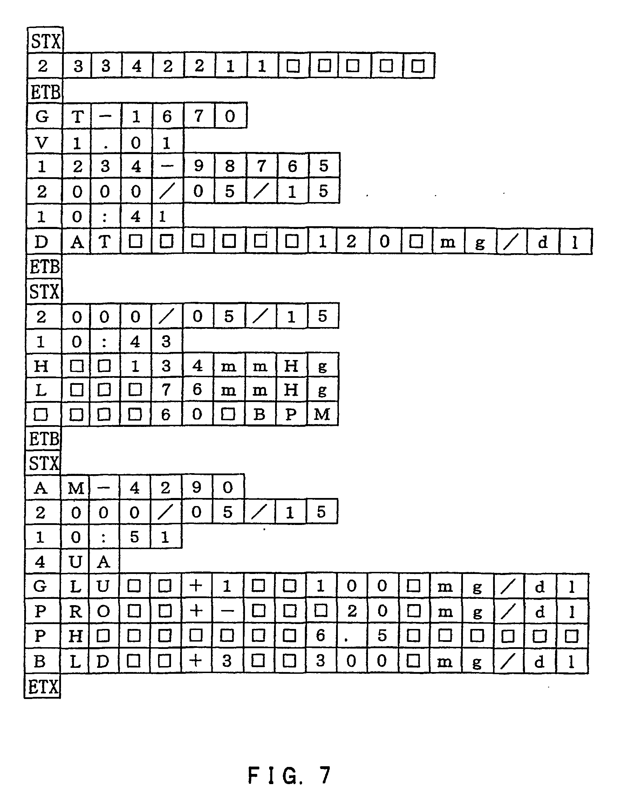

An example of the data that are transmitted from the compact blood glucose measuring device 1 to the data processing device 5 is shown in Fig. 7. Like the other data mentioned before, the data are in a text format and start with the start code "STX". The end of one block is expressed by the block end code "ETB" and the end of the data is expressed by the end code "ETX."

As shown in Fig. 7, for the compact blood glucose measuring device 1, the patient ID (in Fig. 7, 23342211) stored in the ID storage portion 41 is arranged at beginning head of the data (first block) and the measurement data obtained by the compact blood glucose measuring device 1 are arranged in the second block, and the measurement data obtained by the other measurement devices, that is, the sphygmomanometer 6 and the urine analysis device 7, are arranged in order in the subsequent blocks.

The transmission of data from the compact blood glucose measuring device 1 to the data gathering device 5 can be performed after each measurement, however, the compact blood glucose measuring device 1 is capable of storing 120 readings of measured data in the storage portion 4, and thus data also can be transmitted to the data gathering device 5 when 120 readings of measured data have been stored in the storage portion 4.

The user also can bring the compact blood glucose measuring device 1 into which data are stored with him when visiting the hospital and transfer data from the compact blood glucose measuring device 1 to the data gathering device of the medical institution. Accordingly, when making an examination, the physician can confirm the measurement results on the display of the data gathering device, or alternatively can print out the measurement results and confirm them.

It should be noted that this embodiment is not provided for the purpose of limiting the present invention, and various modifications are possible within the scope of the invention.

For example, in the above embodiment, an example was presented in which the measured data obtained by the compact blood glucose measuring device 1 (data measured by the same device) and the measured data obtained by another measuring device such as the sphygmomanometer 6 (data measured by other devices) are transmitted to the data gathering device 5 together, but it is also possible to transmit only the data measured by the same device or only data measured by other devices.

Additionally, in the above embodiment, both the device ID and the patient ID are stored in the ID storage portion 41, and when the measured data are transmitted to the data gathering device, the patient ID is arranged at the beginning of the data, but it is also possible to arrange both the device ID and the patient ID at the beginning of the data, or alternatively, if who the measured data belong to can be assessed accurately on the data gathering side, then it is not absolutely necessary that the measuring device has two types of IDs.

Also, the connection between the measuring device according to the present invention and other measuring devices or the data gathering device is not limited to a wired connection with a cable, and can also be a wireless connection that employs infrared light communication or the like. Additionally, it is not limited to a direct connection, and can be a connection over a LAN or a WAN such as the Internet. Moreover, the data gathering device is not limited to a personal computer, and can also be a server or a host computer.

### Industrial Applicability

A measuring device having an ID for specifying measured persons gathers measured data obtained by other measuring devices and transmits the measured data to the data gathering device after correlating its ID with the measured data. Accordingly, a dedicated device for gathering data is not required, and users can transmit data to the data gathering device simply by connecting each measuring device to a measuring device serving as a main measuring device. As a result, the user comfortably can carry out the measurements required for daily management of his health without having to perform troublesome operations.

## Claims

1. A measuring device comprising:
a storage portion for storing measured results;
a reception portion for receiving data from another measuring device;
a transmission portion for transmitting data to a data gathering device; and
an identifier storage portion for storing an identifier for identifying attributes of measured data;
wherein the storage portion has regions for respectively storing measured data obtained by the measuring device and measured data of the same measured person obtained by another measuring device, and which are received by the reception portion; and
wherein at least one of the measured data obtained by the measuring device and the measured data obtained by another measuring device stored in the storage portion are correlated with at least one identifier stored in the identifier storage portion and are transmitted to the data gathering device from the transmission portion.

2. The measuring device according to claim 1, further comprising a clock portion indicating date and time;
wherein, when the measured data obtained by another device received by the reception portion do not include measured date and time information, information on date and time obtained from the clock portion are correlated with the measured data obtained by the other device, and the measured data are stored in the storage portion.

3. The measuring device according to claim 1 or 2, wherein the identifier stored in the identifier storage portion is at least one of a device identifier, which is uniquely assigned to each measuring device, and a measured person identifier, which is uniquely assigned to each measured person.

4. The measuring device according to any of claims 1 to 3, wherein the measuring device is a blood glucose measuring device.

5. A measured data transmission method, comprising:
gathering, in a main measuring device in which an identifier for identifying a measured person is stored, measured data obtained by another measuring device for that measured person and storing the measured data in a storage portion of the main measuring device; and
correlating the identifier with at least one of measured data obtained by the main measuring device and measured data of the same measured person obtained by another measuring device, and transmitting the measured data from the main measuring device to a data gathering device.

6. The measured data transmission method according to claim 5, wherein, when measured data obtained by the other measuring device do not include information on the measurement date and time, information on date and time is obtained from a clock and correlated with the measured data in question, and the measured data are stored in the storage portion.

7. The measured data transmission method according to claim 5 or 6, wherein the identifier is at least one of a device identifier, which is uniquely assigned to each measuring device, and a measured person identifier, which is uniquely assigned to each measured person.

8. The measured data transmission method according to any of claims 5 to 7, wherein the main measuring device is a blood glucose measuring device.

9. A program that executes, on a computer, a process of:
storing the received measured data, when measured data from another measuring device are received, in a storage portion;
storing the measured data obtained by the measurement, when measurement is performed by a corresponding device, in the storage portion; and
correlating an identifier stored in the corresponding device to at least one of measured data received from the other measuring device and measured data obtained by a measurement of the corresponding device, and transmitting the measured data to a data gathering device.
